(19) Europäisches Patentamt
European Patent Office
Office européen des brevets

(11) Numéro de publication: **0 120 832**
**B1**

(12) # FASCICULE DE BREVET EUROPÉEN

(45) Date de publication du fascicule du brevet: **29.04.87**

(21) Numéro de dépôt: **84870026.6**

(22) Date de dépôt: **22.02.84**

(51) Int. Cl.⁴: **C 07 D 471/16,** A 61 K 31/435 // C07D487/10 ,(C07D471/16, 221:00, 221:00, 209:00)

(54) **Alkyl-4-indolonaphtyridines et compositions pharmaceutiques les contenant.**

(30) Priorité: **25.02.83 LU 84664**

(43) Date de publication de la demande: **03.10.84 Bulletin 84/40**

(45) Mention de la délivrance du brevet: **29.04.87 Bulletin 87/18**

(84) Etats contractants désignés: **BE CH DE FR GB IT LI LU NL SE**

(56) Documents cité:
**EP-A-0 043 811**
**BE-A-870 887**
**BE-A-882 024**
**DE-A-2 736 784**
**US-A-4 190 657**

**Le dossier contient des informations techniques présentées postérieurement au dépôt de la demande et ne figurant pas dans le présent fascicule.**

(73) Titulaire: **OMNICHEM Société anonyme, 12 Avenue de Broqueville, B-1150 Bruxelles (BE)**

(72) Inventeur: **Hannart, Jean, Avenue d'El Pirere, 25, B-1302 Dion Valmont (BE)**

(74) Mandataire: **Van Malderen, Michel, p.a. Freylinger & Associés 22 avenue J.S. Bach (bte 43), B-1080 Bruxelles (BE)**

**EP 0 120 832 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention se rapporte à de nouvelles indolonaphtyridines substituées en position 4 du squelette tétracyclique, à leur mode de préparation et composition pharmacenfigues les contenant.

Ces composés sont susceptibles d'être utilisés pour le traitement des insuffisances cérébrovasculaires, plus particulièrement en gériatrie.

La présente invention concerne plus précisement des derives de l'hexa-hydro-2,3,3a,4,5,6-1H-indolo(3,2,1 de)(1,5) naphtyridine de formule (I)

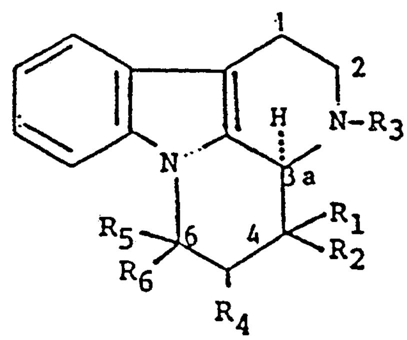

l'un des groupes $R_1$ et $R_2$ représente un groupe alkyle inférieur comportant 1 à 3 atomes de C et l'autre représente un atome d'hydrogène en position "cis" par rapport à l'hydrogène 3a ou, $R_1$ et $R_2$ représentent indépendamment un groupe alkyle comportant 1 à 3 atomes de C ou ensemble un groupe alkanediyl comportant de 4 à 6 atomes de carbone,

$R_3$ représente un groupe alkyle comportant 1 à 3 atomes de C, un atome d'hydrogène ou un groupe benzyle et soit,

$R_5$ représente un groupe carboalkoxy comportant 2 à 4 atomes de C ou un atome d'hydrogène et $R_6$ représente avec $R_4$ une liaison carbone-carbone supplémentaire soit

$R_4$ représente un atome d'hydrogène et $R_6$ et $R_5$ représentent ensemble un atome d'oxygène ou respectivement un atome d'hydrogène et un groupe hydroxy,

Toutes les formes stéreochimiques possibles des composés de formule (I) sont comprises dans la présente invention. En particulier, ces composés peuvent se présenter sous formé d'isomères optiques ou de mélange racémique.

Le squelette polycyclique des dérivés de formule (I) correspond à celui de la vincamine dans lequel le cycle D est absent. Ce squelette est par ailleurs identique à celui des canthinones-6 qui forment une classe d'alcaloides naturels (voir par exemple L.A.Mitscher et al, Heterocycles, 3, 7, 1975).

On peut donc décrire les composés de l'invention comme étant des dérivés de canthinones, de canthinols ou de canthènes lorsque respectivement R5 et R6 représentent dans la formule (I) un atome d'oxygène. R5 représente un groupe hydroxy et R6 un atome dihydrogène ou R6 représente un atome d'hydrogène et R5 et R4, ensemble, une liaison C-C supplémentaire. Le squelette hexahydro-1,2,3,3a,4,5-canthinone-6 correspond donc au squelette dexahydro 2,3,3a,4,5,6, oxo-6- 1H indolo(3,2,1-de)(1,5)naphtyridine.

Le BE-A 853.435 divulgue l'obtention d'un nombre important d'indolonaphtyridines diversement substituées. Parmi ces dernières on relèvera en particulier des carboalkoxy-4-hexahydroindolonaphtyridines.

Le BE-A- 870.887 décrit des composés de formule (I) dans laquelle R1=R2=H, R5 est un groupe carbométhoxy et R4 et R6 forment ensemble une liaison carbone-carbone supplémentaire. On les a appelés chanodéséthylapovincamines ou "petites apovincamines". De nombreux dérivés analogues sont décrits dans le BE-A- 884.145.

Le BE-A- 857.816 décrit et revendique des composés de formule (I) dans lesquels R1 et R2 représentent un atome d'hydrogène et R5 et R6 représentent ensemble un atome d'oxygène. Il s'agit alors de dérivés d'hexahydrocanthinones-6.

Les brevets belges BE-A- 816.759 et BE-A- 882.024 décrivent l'obtention de seco-D-homo-E-vincamones (appelées aussi chano-D-homovincamones) substitués par un groupe éthyle à la position equivalente à la position 4 de la formule (I).

Le brevet EP-A- 43 811 et le brevet des US-A- 4 190 657 décrivent des dérivés du type canthène dans lesquels $R_1$ et $R_2$ représentent un atome d'hydrogène, $R_4$ et $R_6$ forment ensemble une liaison carbone-carbone.

La demanderesse a maintenant constaté que des dérivés plus actifs peuvent être obtenus en placant un ou deux substituants alkyle inférieur en position 4 du squelette hexahydro 2,3,3a,4,5,6-1H- indolo (3,2,1-de)(1,5)naphtyridine.

Les composés préférés sont ceux dans lesquels R1 et R3 représentent un groupe éthyle et R2 représente un

atome d'hydrogène. Quelques composés plus particulièrement préférés de ce groupe sont

- la diéthyl-3.4 bêta- hexahydro-1,2,3,4,5-canthinone-6 et son isomère optique (1,$R_1$ = $R_3$=éthyle, $R_2$ = $R_4$ = H, $R_6$, $R_5$ = O), sous la forme racémique In
- la diéthyl-3,4 bêta-tétrahydro-2,3,3a,4 1H indolo (3,2,1-de)(1,5) naphtyridine-6-carboxylate de méthyle (1, $R_1$ = $R_3$ = éthyle $R_2$ = H, $R_5$, $R_4$ = liaison, $R_6$ = $CO_2CH_3$ et son isomère optique (exemple 20), sous la forme racémique Is.

La triéthyl-N,4,4-hexahydro-1,2,3,3a,4,5-canthinone-6 s'est également rélévée particulièrement active.

Pour les dérivés monosubstitués en position 4, il a aussi été constaté que de manière générale un des isomères de position en 4 présente un profil pharmacologique plus attrayant que l'autre. En série canthinone, on a attribué, sur la base des spectres de résonance magnétique nucléaire, la stéréochimie H3a-H4 cis au dérivé le plus actif (constante de couplage H-H). Par ailleurs une différence de polarité importante peut être observée. En particulier In, comparé à son isomère, est moins polaire en chromatographie sur couche mince (ccm, silica gel éluant dichlorométhane méthanol 98:2, v:v), polarité estimée sur la base de son Rf qui est supérieur.

De manière générale les composés de l'invention présentent des toxicités plus faibles que celles qui caractérisent des composés de référence comme la vincamine ou la vincamone. On observe également une activité antianoxique remarquable et inattendue et une bonne activité débitmétrique et oxymétrique. On constate par ailleurs que certains problèmes de résorption rencontrées avec des molécules de l'art antérieur pharmacologiquement actives ne se présentent plus.

Plusieurs voies de synthèses, dont certaines sont originales, ont été mises au point pour l'obtention des dérivés décrits dans la présente invention.

Une de ces voies fait intervenir une condensation d'une tryptamine et d'un aldéhyde ou d'un chlorure d'acide fonctionnalisé, (condensation de Pictet-Spengler ou de Bischler), suivie éventuellement d'une modification chimique classique (déshydratation, réduction etc...) Les synthons aldèhydes fonctionnalisés doivent être prèalablement obtenus. (Voir p.e. composè 8 dans Szantav et al, Heterocycles 6, 1149, 1977). Ces synthèses sont analogues à celles décrites dans les brevets belges BE-A- 870.887 et BE-A- 884.145.

Dans le cas particulier des dérivés éthyl -4- hexahydro- propyl-4-canthinones-6, on peut effectuer une réaction de Emde au départ de la déhydro-14,15-vincamone. L'alkylation de l'azote beta de cette vincamone suivie d'une hydrogénation catalytique conduit à la rupture du cycle D pour fournir les composés désirés. L'agent alkylant utilisé est de préference l'iodure de méthyle ou l'iodure d'éthyle et le catalyseur utilisé sera par exemple l'oxyde de platine.

Une autre voie de synthèse, de canthinols ou de canthènes, fait intervenir une régression de cycle des composés homo-E correspondants de formule (II)

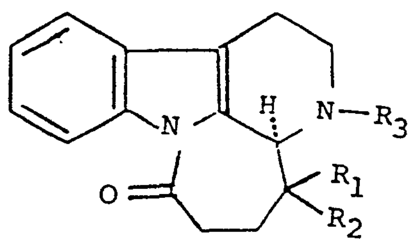

(voir BE-A- 816.759) via un intermédiaire de formule (II)

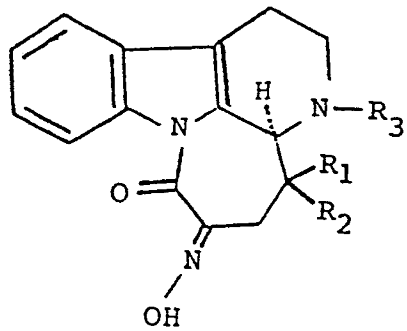

La régression de cycle est obtenue par oxydation de l'homocanthinone (II) (une azépino 1,2,3 - 1,m- bêta carboline) pour fournir l'oxyimino-homocanthinone (III) correspondante. Cette oxydation est effectuée par un nitrite organique, par exemple le t-butylnitrite, ou le nitrite d'isoamyle, en présence d'une base, de preférence un alcoolate, dans un solvant organique.

L'hydroxyiminohomocanthinone conduit à la canthinone simple par chauffage en milieu alcalin protique (p.e. soude-alcool) pendant plusieurs heures suivi par traitement en milieu acide.

Par ailleurs, la "petite vincamine" correspondante peut être ainsi obtenue à partir de l'oxo-7-hydroxyimino-6-homocanthane. Il y a alors lieu de chauffer ce dernier composé par exemple en solution dans un mélange eau-méthanol- dioxanne, en présence de bisulfite de sodium ou $NaHSO_3$. On obtient ainsi les deux isomères en position 6 (hydroxy alpha, hydroxy bêta) qui peuvent être séparés par recristallisation (voir par exemple Ir'a et Ir'b de l'exemple 24).

Enfin, il a été constaté que les pyrrolo (2,3-d) carbazoles de formule (IV)

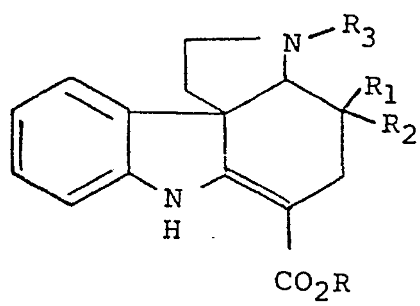

peuvent, en milieu acide en présence d'un agent d'oxydation, se réarranger pour fournir les "petites vincamines" correspondantes, c'est à dire des composés de formule I dans lesquels R5 et R6 représentent respectivement un groupe carboalkoxy et un g roupe hydroxyle et R4 représente un atome d'hydrogène. Ce réarrangement est analogue à celui qui a été observé pour la vincadifformine et qui conduit avec d'excellents rendements à la vincamine (voir BE-A- 848.475 et BE-A- 761.628). L'agent d'oxydation utilisé pour provoquer le réarrangement est de préférence un peracide organique tel l'acide métachloroperbenzoïque. D'autres peracides peuvent être utilisés par exemple l'acide permaléique ou l'acide perphtalique. L'agent d'oxydation peut également être le peroxyde d'hydrogène éventuellement en présence d'un catalyseur à base de métal tel le cuivre ou le molybdène. Les "petites vincamines" conduisent par déshydratation aux "petites apovincamines" de l'invention, par exemple par distillation azéotropique en présence d'un catalyseur acide tel l'acide paratoluène sulfonique. Certaines "pétites vincamines" classiques ou "chanovincamines" ont été décrites par Le Men et al, Bull. Soc. Chimique de France, 1207, 1977.

Les meilleurs rendements pour le réarrangement susmentionné ont été observés avec des composés disubstitués en position 4 du squelette pyrrolo(2,3-d)carbazole.

Les pyrrolo (2,3-d) carbazole -6- carboxylates de formule (IV) ou "seco-D-vincadifformines" sont obtenues à partir d'azépino(4,5-b)-indoles. Ces composés et leur mode d'obtention sont décrits dans la EP-A- 64.317.

On peut également obtenir par cette voie les "petites vincamones" (hexahydrocanthinones), les canthinols et les canthènes correspondants. Il y a lieu alors de faire réagir la "petite vincamine" en présence d'un agent d'oxydation adéquat comme décrit dans la littérature pour les composés du type "vincamine" (voir p.e. BE-A-799,679, Richter).

L'hexahydrocanthinone isolée peut être réduite pour fournir le canthinol correspondant qui peut être à son

tour déshydraté pour fournir le canthène correspondant.

La réduction est effectuée de préférence avec LiAlH4 dans le THF à une température comprise entre -10°e et 0°C. D'autres hydrures métalliques peuvent être utilisés. La déshydratation est effectuée de manière classique par distillation azéotropique en présence d'acide paratoluènesulfonique.

Les déplacements chimiques des spectres RMN sont exprimés en ppm par rapport au tétraméthylsilane. Les abbréviations ou symboles suivants sont utilisés: s = singulet, bs = singulet large, m = multiplet, t = triplet.

Les dérivés numerotés en prime sont des diastéréoisoméres des dérivés correspondants sans prime, l'isomérie se situant au niveau du groupe éthyle en position 4 par rapport à l'hydrogène en position 3a.

**Exemple 1**

Benzyl-3-diéthyl-4,4-hexahydro-1,2,3,3a,4,5-7H-pyrrolo(2,3-d) carbazole-6-carboxylate de méthyle (séco D vincadifformine IVa) (Produit intermédiare)

a) méthano-azépinoindole

Une solution de 7 g de methyl hexahydro- 1,2,3,4,5,6-azepino (4,5-b) indole -5- carboxylate (28,6 mmol) et de 3,43 g (34,3 mmol) d'éthyl-2-butyraldéhyde et de 0,1 g d'acide benzoïque dans 100 mL de CH3OH est agitée 24 h à température ambiante. On isole ainsi de manière classique environ 10 g de méthanoazépinoindole (voir demande de brevet européen 64.317).

b) seco-D vincadifformine (IVa)

La méthanoazépinoindole obtenue en a, 5,33 g de bromure de benzyle (31,1 mmol), 7 mL de diisopropylethylamine en solution dans 200 mL de CHCl3 sont portés à reflux pendant 6 jours. Après évaporation du solvant, on traite le résidu par 50 mL de CH3OH, 200 mL d'une solution aqueuse saturée en K2CO3 et 200 mL d'éther. On sépare la phase organique. La phase aqueuse est extraite 3 x à l'éther. Les phases organiques sont réunies, lavées et séchées. On obtient 12,16 g de matière organique qu'on purifie par chromatographie sur SiO2 (élution CH2Cl2/CH3OH 2%) et par cristallisation dans le CH3OH. On isole ainsi 9,86 g du compose (IVa) (rendement 83%).

Fusion 111°C

Spectre de masse: 416 (10%, M+), 332 (8), 202 (100), (38) Spectre infra-rouge (3% CC14) 3385, 2964, 2880, 1680, 1621, 1610, 1462, 1435, 1247 $cm^{-1}$.

Spectre ultra-violet (CH3OH, λ max, nm, log ε): 331 (4,26), 302 (4,08), 229 (4,03)

Spectre de RMN (CDCl3): 9,03 (bs, 1H), 7,70-6,60 (m,9H), 4,33 (d, 1H, J=13 Hz), 3,93-3,46 (m,4H) 3,23-1 33(m,9H)

**Exemple 2**

Benzyl-3-diéthyl-4,4-hydroxy-6-hexahydro-2,3,3a,4,5,6-1H-indolo-(3,2,1-de)(1,5)naphtyridine-6-carboxylate de méthyle Ia (Produit intermédiaire)

On prépare une solution de 10 g (24 mmol) de séco-D vincadifformine (IVa) dans 225 mL de méthanol contenant 0,96 g d'HCl (pH 1). Après addition de 4,84 g (24 mmol) diacide métachloroperbenzoïque à 85% (MCPB), le mélange réactionnel est agité 3 heures à temperature ambiante. On ajoute 1,5 g de MCPB et on poursuit liagitation 10 heures. Du CH3OH saturé en NH3 est ajouté à la solution jusqu'à pH basique. Après évaporation du solvant, le résidu est traité par 350 mL de CH2Cl2, 150 mL d'eau et 150 mL d'une solution saturée en K2CO3. La phase organique est décantée. La phase aqueuse est extraite 2x par du CH2Cl2. Les phases organiques réunies sont lavées par 2x 200 mL d'eau distillée et par 1x 200 ma d'une solution aqueuse saturée en NaCl. Après séchage sur Na2SO4. filtration et évaporation, on obtient 12,70 de matière organique qu'on purifie par chromatographie sur colonne de SiO2 (elution: CH2Cl2) et par cristallisation dans le méthanol. On isole ainsi 6 g de la séco-D vincamine Ia (1 seul isomère visible, rendement 57,9%).

Fusion 151,5°C

Spectre UV (max, CH3OH): 276 nm

Spectre IR (CCl4, 3%) 3520, 3022, 2960, 2880, 1736, 1455, 1247 $cm^{-1}$

Spectre RMN (CDCl3): 7,53-6,76 (m,9H), 4,30(s,1H), 4,00-3,56(m,4H), 3,13-1,90(m,6H). 1,15-0,60(m,6H)

**Exemple 3**

Benzyl-3-spirocyclohexyl-4-hydroxy-6-hexahydro-2,3,3a,4,5,6-1H-indolo(3,2,1-de) (1,5)naphtyridine-6-carboxylate de méthyle (Ib) (Produit intermédiaire)

On prépare une suspension de 24,4 g (57 mmol) de la séco-D vincadifformine correspondante (IVb) (exemple 22 EP-A- 64.317) dans 700 mL de CH3OH chlorhydrique à 0,5%. On ajoute 14,3 g (0,07 M) de MCPB et on agite 2 heure à température ambiante. Après addition de CH3OH saturé en NH3, la solution est évaporée sous vide. Le

résidu est réparti entre 300 mL de CH2Cl2 et 300 mL d'une solution aqueuse 1M de Na2CO3. La phase organique est decantée et la phase aqueuse est extraite 2 x au CH2Cl2. Les phases organiques réunies sont lavées à l'eau distillée et par une solution aqueuse saturée en NaCl. Elles sont séchées sur Na2SO4. Après filtration et évaporation on isole ainsi 23 g de matière organique. Par cristallisation dans le CH3OH, on obtient 11,33 g de la seco-D vincamine Ib, Le residu de cristallisation purifié par chromatographie sur SiO2 (élution: CH2Cl2) permet encore d'isoler 1 g de (Ib) (rendement 49%).

Fusion 200° C

Spectre de masse: 444 (M+,16%), 348(10), 336(59), 253(51), 247(66), 232(15), 222(26), 207(20), 170(35), 144(61).

Spectre UV (CH3OH): max 276 nm

Spectre IR (KBr 1%) 3500, 3030, 2935, 2860, 1737, 1459, 1437, 1242 $cm^{-1}$

Spectre RMN (COCl3) 7,63-6.96 (m,9H), 4,36(s,1H), 4,03-3.076 (m,5H) dont 3,86(s,3H), 3,43(s,1H), 3,16-1,06 (m,16H)

**Exemple 4**

Diéthyl-4,4-hydroxy-6-hexahydro-2,3,3a,4,5,6-1H-indolo-(3,2,1-de)(1,5)naphtyridine-6-carboxylate de méthyle (Ic) (Produit intermédiaire)

L'hydrogénation à pression atmosphérique d'une solution de 9 g (20,8 mmol) de la séco-D-vincamine la dans 180 mL d'acide acétique en présence de 1,4 g de charbon palladié 10% est stoppée après 15 heures. La solution est filtrée sur décalite. le filtre est rincé par 80 mL de méthanol. Le filtrat est versé dans 300 mL de CH2Cl2 et est alcalinisé par addition goutte à goutte d'une solution aqueuse saturée en K2CO3. La phase organique est décantée et la phase aqueuse extraite 2x au CH2Cl2. Les phases organiques rassemblées sont lavees à l'eau puls par une solution aqueuse saturée en NaCl. Elles sont séchées sur Na2SO4. Après filtration et évaporation on obtient 7,2 g d'une poudre blanche qui fournit après cristallisation, dans le CH3OH 6,5 g de la séco-D-vincamine (Ic) (rendement 91 3%)

Fusion 174,7° C

Spectre de masse: 342(M+,49%), 313(13), 258(18), 198(24), 170(100)

Spectre IR (CHCl3): 3520, 2958, 1731, 1453. 1250 $cm^{-1}$

Spectre UV: 281 nm, log ε:3,91

Spectre RMN (CDCl3) 7,46-6,80 (m,4H), 4,53 (bs,1H), 3,93 (s,1H), 3,76(s,3H), 3,60-2,36 (m,5H), 2,10 (d,2H), 1,90-0,56 (m,10H)

**Exemple 5**

Spirocyclohexyl-4-hydroxy-6-hexahydro-2,3,3a,4,5,6-1H-indolo(3,2,1-de)naphtyridine-6-carboxylate de méthyle (Id) (Produit intermédiaire)

L'hydrogénation à pression atmosphérique d'une solution de 6,66 g de la seco-D-vincamine Ib (15 mmol) dans 140 mL diacide acétique glacial en présence de 1,20 g de charbon palladié à 10% est stoppée après 20 heures. La solution est filtrée sur décalite. Le filtre est rincé par 50 ml de méthanol. Le filtrat est versé dans 250 mL de CH2Cl2 et est alcalinisé par addition d'une solution aqueuse saturée en K2CO3. La phase organique est décantée et la phase aqueuse extraite 2 x au CH2Cl2. Les phases organiques réunies sont lavées à l'eau puis par une sulution aqueuse saturée en NaCl. Après séchage sur Na2SO4, filtration et évaporation sous pression réduite, on obtient 5,39 g d'une poudre qui fournit après cristallisation dans le CH3OH 4,33 g de la séco-vincamine Id (rendement 81,5%).

Fusion: 232-235° C (décomposition)

Spectre UV (CH3OH) 281 nm (3,93)

Spectre IR (KBr 1%) 3450, 2940, 1740, 1452, 1428, 1198 $cm^{-1}$

Spectre de masse: 354 (M+, 98%), 336(10), 325(27), 295(15), 266(17), 252(27),198(26),170(100)

Spectre de RMN (COCl3) 7,63-6,90 (m,4H), 3,81(s,3H), 3,70(m,1H), 3,56-2,46(m,5H),2,30-1,03(m,12H).

**Exemple 6**

Diéthyl-4,4-tétrahydro-2,3,3a,4-1H-indolo(3,2,1-de)(1,5)napthyridine-6-carboxylate de méthyle (Ie)

Une suspension de 6 g (17,5 mmol) de la séco-D-vincamine Ic et de 6 g d'acide paratoluénesulfonique (34,8 mmol) dans 650 mL de benzène sec est portée à reflux dans un ballon de 1 L muni d'un Dean-Stark. Après 5 h de reflux, la solution refroidie est versée dans 300 mL d'une solution aqueuse de Na2CO3 1M. Après 15 minutes d'agitation, le mélange est décanté. La phase aqueuse est extraite par 3 x 100 mL de benzène. Les phases organiques réunies sont lavées à l'eau par une solution aqueuse saturée en NaCl. Après séchage et évaporation sous pression réduite, on obtient 5,95 g de matière organique. Une cristallisation dans le méthanol

fournit 5,65 g de la seco-D-apovincamine Ie (rendement 99%).
Fusion: 99° C
Spectre de masse m/e 324(M+,100%), 309(83), 280(7), 266(14), 170(54) pour C2OH24N2O2.
Spectre IR: (CCl4, 3%) 2960, 1734, 1738, 1609, 1453, 1440, 1253 $cm^{-1}$
Spectre UV: (CH3OH, 10 mg/L, λ mx) 275 (4,039, 313 (3,73).
Spectre RMN (CDCl3) 7,63-686 (m,4H) 6,10(s,1H), 4,15(m,1H), 3,93(s,1H), 3,6-2,53(m,4H), 1,93-0,36 (m,11H) dont 0,58 (t) et 1,00(t).

**Exemple 7**

Spirocyclohexyl-4-tetrahydro-2,3,3a,4,-1H-indolo(3,2,1-de) (1,5) naphtyridine-6-carboxylate de méthyle (If).

Une suspension de 7,83 g de seco-D-vincamine Id (22,1 mmol), de 7,80 g d'acide paratoluénesulfonique (41 mmol) dans 850 mL de benzène est portée à reflux 5 heures dans un ballon muni d'un Dean-Stark. Après refroidissement la solution obtenue est versée dans 400 mL d'une solution aqueuse 1M en Na2CO3. Après 30 minutes d'agitation la phase organique est séparée, la phase aqueuse est extraite par 3x 100 mL de benzène. Les phases organiques réunies sont lavées à l'eau puis par une solution saturée en NaCl. Après séchage sur Na2SO4, filtration, évaporation et cristallisation dans le méthanol, on isole 6,77 g de séco-D-apovincamine If (rendement: 91 %).
Fusion 132-134° C
Spectre IR: (KBr, 1%) 3440, 2932, 2923, 2858, 1727, 1643, 1604, 1450, 1295, 1253 $cm^{-1}$
Spectre UV (CH3OH, λ max, log ε): 315 (3,75), 275 (4,06)
Spectre RMN (CDC13,ppm): 7,53-6,86 (m,4H), 6,45 (s,1H), 3,90 (s,3H), 3,73 (m,1H), 3,51, 2,46 (m,4H), 1,96-0,70 (m,11H).

**Exemple 8**

Triéthyl-3,4,4-tétrahydro-2,3,3a,4-1H-indoio(3,2,1-de)(1,5) naphtyridine-6-carboxylate de méthyle (Ig)

Une suspension de 6 g (16,4 mmol) de séco-D-apovincamine Ie, de 3,91 g de Na2CO3 (36,9 mmol) et de 6,61 g d'iodure d'éthyle (55,2 mmol) dans 150 mL d'éthylméthylcétone est portée à reflux 24 heures. Après addition de 3 g d'iodure d'éthyle supplémentaire, le reflux est encore poursuivi pendant 24 heures. Après refroidissement, le mélange réactionnel est filtré puis concentré sous pression réduite. Le résidu est réparti entre 200 mL d'éther et 100 mL d'eau. La phase aqueuse est décantée et est extraite 2x par de l'éther. Les phases organiques sont réunies, lavées par H2O et par NaCl sat. Après séchage et évaporation on obtient 6 g de matière organique qu'on purifie par chromatographie sur colonne de SiO2 (élution CH2Cl2) puis par cristallisation dans le méthanol. On isole ainsi 4.5 g de seco-D-apovincamine Ig unitache en ccm (rendement 69,5%).
Fusion 73-75° C
Spectre de masse: 352 (M+, 100%), 337(99), 323 (10), 198 (68) pour C22 H28 N2 O2.
Spectre IR (CCl4, 3%): 2973, 1731, 1632, 1608, 1453, 1252, 1197 $cm^{-1}$
Spectre UV (CH3OH, max, log ε): 274(4,07), 313 (3,75)
Spectre RMN (CDCl3, ppm): 7,53-6,83 (m,4H), 6,06(s,1H), 3,90(s,3H), 3,63(bs,1H), 3,33-2,30(m,6H), 1,66(t,3H), 1,13(t,3H), 0,56(t,3H).

**Exemple 9**

Ethyl-3-spirocyclohexyl-4-tétrahydro-2,3,3a,4-1H-indolo(3,2,1-de)(1,5)naphtyridine-6-carboxylate de méthyle (Ih).

En appliquant un mode opératoire analogue à celui décrit dans l'exemple précédent on prépare le composé Ih à partir du composé (If).
Spectre de masse 364 (M+,30%) pour C23 H28 N2 O2
Spectre IR (KBr,1%): 2937, 1730, 1633, 1600, 1490 $cm^{-1}$
Spectre UV (CH3OH, max): 270, 315 nm
Spectre RMN (CDCl3, ppm): 7,46-6,36(m,4H), 6,43(s,1H), 3,30(s,3H), 3,26(bs,1H), 3,13-2,46(m,6H), 2,00-0,76(m,13H).

**Exemple 10**

Benzyl-3-diéthyl-4,4-hexahydro-1,2,3,3a,4,5-6H-canthinone-6 Ii

Une suspension de 13.82 g (32 mmol) de séco-D-vincamine Ia dans 375 mL d'une solution aqueuse 0,28 M de KOH est portée à reflux 5 heures. La solution refroidie à +/- 10°C est filtrée sur décalite. Le filtre est rincé par 70 mL d'eau. Après addition de 23,5g de K3(Fe(CN)6), la solution aqueuse est agitée 48 heures à température normale. On ajoute 500 mL d'eau et 500 mL de CH2Cl2. Les phases sont décantées et la phase aqueuse est extraite 3x par du CH2Cl2. Les phases organiques rassemblées sont lavées à l'eau puis par une solution saturée en NaCl. Après séchage, filtration et concentration sous vide, on obtient une poudre que l'on recristallise dans le CH3OH. On obtient ainsi 9,80 g de l'hexahydrocanthinone Ii (rendement: 62,3%)

Fusion 172,6°C

Spectre UV (CH3OH, max, logε): 305 (3,88), 266 (4,16), 241 (4,46)

Spectre IR (KBr, 1%): 2964, 1704, 1632, 1456, 1370, 1334, 1140 $cm^{-1}$

Spectre RMN (CDCl3, ppm): 8,40(1H,m), 3,93 (s,2H), 3,76 (s,1H), 3,26-2,16 (m,6H), 2,03-0,53 (m,10H).

**Exemple 11**

Diéthyl-4,4 hexahydro 1,2,3,3a,4,5 canthinone-6; Ij.

L'hydrogénation à pression atmosphérique d'une solution de 1.5 g (4,03 mmol) de l'hexahydrocanthinone Ii (exemple 10) dans 40 mL d'acide acétique glacial et dans 5 mL de $CH_3OH$-acide chlorhydrique à %, en présence de 0,4 g de C/Pd à 10 % est stoppée après 5 heures. Après addition de 10 ml de $CH_3OH$, on chauffe la solution pour dissoudre le précipité formé. La solution est filtrée à chaud sur décalite. Le filtre est lavé au méthanol. Les solvants sont évaporés sous pression réduite. Le résidu obtenu est réparti entre 50 mL de $CH_2Cl_2$ et 50 mL d'une solution aqueuse saturée en $K_2CO_3$. Après 15 minutes d'agitation, les phases sont séparées. La phase aqueuse est extraite 2 x par du $CH_2Cl_2$. Les phases organiques rassemblées sont lavées à l'eau et par une solution aqueuse saturée en NaCl. Elles sont séchées sur $Na_2SO_4$. Après filtration, évaporation, on obtient 1,00 g de matière organique qui fournit après cristallisation dans le $CH_3OH$, 0.9 g de canthinone Ij (rendement: 79%).

Fusion: 113°C

Spectre UV: $CH_3OH$ 295 (3.66), 268 (4.01) 242 (4.30)

Spectre IR ($CCl_4$ 3%): 3055, 2970, 1708, 1630, 1450, 1376, 1332, 1133 $cm^{-1}$

Spectre RMN ($CDCl_3$): 8,26 (m,1H), 7,40-6,96 (m,3H) 3,86 (m,1H) 3,63-2,33 (m,7H) dont 2,50 (s;2H), 1,73 - 0,50 (m,10H).

**Exemple 12**

Triéthyl-3,4,4 hexahydro-1,2,3,3a,4,5-canthinone-6 Ik.

On porte au reflux une solution de 26.5 g (94 mmol) de, la canthinone Ij et de 73,2 g (0,469 mol) d'iodure d'éthyle dans 350 mL d'éthylméthylcetone contenant 19.92 gr de $Na_2CO_3$.

Après 5 jours de reflux, le solvant est évaporé sous vide.

Le résidu est reporté entre 300 ml de $CH_2Cl_2$ et 200 ml d'eau distillée.

La phase organique est lavée par une solution aqueuse saturée en NaCl et est séchée sur $MgSO_4$. Après évaporation du solvent, le résidu est purifié par chromatographie sur colonne de $SiO_2$ (élution $CH_2Cl_2$ / $CH_3OH$ 98.5/1.5) on obtient 21 g de Ik sous forme d'une huile amorphe (rendement- = 72%).

Fusion (chlorhydrate): 197-200°C (décomp.)

Spectre de Masse: 310 (M+ 70), 281 (44) 198(100)

Spectre UV: ($CH_3OH$) 242, 267, 295.

Spectre. IR: ($CCl_4$ 3%) 2968, 1709, 1624, 1453, 1364, 1330 cm.

Spectre de RMN $CDCl_3$: 8,20 (m,1H), 7,40-6,93 (m,3H) 3,46 (bs,1H) 3,21-2,16 (m,8H) dont 2,45 (s, 2H), 1,73-0,50 (m).

**Exemple 13**

Spirocyclohexyl-4-éthyl-3-hexahydro-1,2,3,3a,4,5-canthinone-6 (Il)

Selon un procédé analogue à celui décrit pour (Ij) et (Ik) on prépare (Il).

Fusion: 144,8°C

Spectre de Masse: 322 (M+) pour C21 H26 N2 O

Spectre IR: KBr 1%, 2970, 2935, 2860, 1703, 1622, 1453, 1380 $cm^{-1}$.

Spectre UV ($CH_3OH$): 243, 266, 293 nm

Spectre RMN ($CDCl_3$, ppm): 8,26 (m,1H), 7,56-7,00 (m,3H) 3,26 (ms,1H), 3,20-2,33 (m, 8H) 2,03-0,86 (m, 13H)

**Exemple 14**

Diéthyl-3,4-octahydro-1,2,3,3a,4,5,6,7-oxo-7-oxyimino-6-azepino (1,2,3-lm)-β-carboline (III) (Produit intermédiaire)

A une solution de 15 g (0 05 mol) de chano-E-homovincamone II (isomère le plus polaire $R_1 = R_3 =$ éthyle, $R_2 =$ H, voir brevet belge 882.024) dans, 300 mL de toluène sec on ajoute 64,9 mL de tert-butylnitrite (0,56 mol) puis une solution de 0,0828 mole de tert-amylate de sodium dans 150 ml de toluène et on laisse sous agitation pendant 90 minutes.

Le milieu réactionnel est ensuite versé sur 500 mL d'une solution aqueuse contenant 10 % de chlorure d'ammonium et la phase organique est decantée puis lavée, séchée sur $MgSO_4$ et évaporée à sec.

On obtient 16 g de résidu solide unitache en ccm $SiO_2$ ($CH_2Cl_2$ + 5 % MeOH). Le chlorhydrate du produit désiré est obtenu par dissolution des 16 g dans l'acétone et addition d'éther HCl jusqu'à pH acide. On obtient ainsi 11 g de cristaux blancs.

Fusion du produit chlorhydrate: 236° C

Spectre RMN (base, $CDCl_3$): 8,4 ppm (massif: 1H) 7,4-7ppm (massif 3H) 3,9 ppm (doublet 1H J 3a-4; 5cps 1,3 - 0,8 ppm (massif 6H).

Spectre IR du produit base (film) ($cm^{-1}$) 3400, 2970, 1685, 1610, 1450, 1370, 1330, 910, 730.

Spectre UV du produit chlorhydrate (c = $2,73.10^{-5}$ mol/L MeOH) λ max (logε): 257 (4,26) 308 (3,69) nm.

Spectre de masse (m/e): 325, 308, 296, 283, 268, 252, 239, 226, 214, 199, 198, 180, 167 pour C19 H23 N3 O2.

**Exemple 15**

Diéthyl-3,4-hexahydro-2,3 3a,4,5,6-oxo-6-1H-indolo(3,2,1-de)(1,5)-naphtyridine (In).

9,45 g (29 mmol) de diéthyl -3,4- octahydro -1,2,3,4,5,6,7- oxo-7 -oxyimino6-azepino(1,2,3-lm) carboline sont mis en suspension dans 40 mL d'éthoxyéthanol. On ajoute 2,32 g (58 mmol) de soude et le milieu réactionnel est agité 1 h à température ambiante puis 18 h à reflux. Le milieu réactionnel est alors évaporé à sec et le résidu est repris par 50 mL HCl 3N à reflux pendant 1 h. La solution est alors refroidie, basifiée et extraite au $CH_2Cl_2$. La phase organique est décantée et agitée pendant 15 min en présence de 100 g de $Al_2O_3$. Après filtration de l'alumine, le filtrat est lavé à l'eau, séché et évaporé. On obtient 6 g de résidu solide qui est dissous dans l'acétone et dont on fait le chlorhydrate par addition d'éther saturé en acide chlorhydrique. On filtre ainsi 4 g de cristaux blancs.

Le composé In s'est révélé moins polaire en ccm lorsqu'il est comparé avec son isomère en position 4 (composé In' de l'exemple 21).

Fusion: 240-241° C (chlorhydrate)

Spectre UV (chlorhydrate, c = $3,14.10^{-5}$ mol/L, MeOH) max, log ε 242(4,29), 2,65(3,99), 296-303(3,59)

Spectre IR (base, film, $cm^{-1}$): 2960, 1700, 1635, 1450, 1380, 1320, 1150, 910, 750

Spectre de masse: m/e 282, 281, 267, 253, 225, 198, 197, 196, 182, 167, 154, 142 pour C18 H22 N2 O.

Spectre RMN (CDCl3): 8,3 (massif 1H), 7,4-7,1(massif 3H), 1,3-0,8 (massif 6H)

**Exemple 16**

Diéthyl-3,4-hexahydro-2,3,3a,4,5,6-hydroxy-6-1H-indolo(3,2,1-de) (1,5)naphtyridine (Io) (canthinol).

2,61 g (69 mmol) de $LiAlH_4$ sont mis en suspension dans 86 mL de THF sec et la suspension est refroidie à -5°C. Une solution de 14,54 g (51 mmol) de diéthyl-3,4-hexahydro 2,3,3a,4,5,6 -oxo-6 1H indolo(3,2,1-de) (1,5) naphtyridine In dans 190 mL de THF sec est alors ajoutée goutte à goutte en évitant que la température monte au dessus de 0°C. 30 min après la fin de l'addition, l'excès de $LiAlH_4$ est détruit par addition lente de THF humide. Les sels sont filtrés sur décalite et le filtrat est évaporé à sec. Le résidu est repris par la chlorure de méthylène et extrait par une solution de $H_2SO_4$ 2%. La phase aqueuse acide est ensuite basifiée et extraite au $CH_2Cl_2$. La phase organique est lavée, séchée et évaporé et le résidu est cristallisé dans l'acétone. On obtient 12 g (42 mmol) d'un produit unitache en ccm ($SiO_2$, $CH_2Cl_2$-5%MeOH). Rendement: 82 %.

Fusion: 176-178°C

Spectre UV (c = 3,54 $10^{-5}$ mol/L, MeOH, log ε): 228(4,52), 2,81 (3,86), 290 (épaulement, 3,74) nm

Spectre IR (KBr, $cm^{-1}$) 3200, 2800, 1460, 1190, 1050, 740.

Spectre de masse: 284 M+ pour $C_{18}H_{24}N_2O$

Spectre RMN (base, DMSO-d6): 7,6-6,9 (massif 4H), 6,3 (massif 1H), 6,00(1H), 1,3-0,9 (massif, 6H).

**Exemple 17**

Diéthyl-3,4-tétrahydro-2,3,3a,4-1H-indolo(3,2,1-de)(1,5)naphtyridine (Ip) (canthène).

5,85 g (0,02 mol) de diéthyl-3,4-hexahydro-2,3,3a,4,5,6-hydroxy-6 6H indolo(3,2,1,-de)(1,5) naphtyridine (obtenus à partir de l'isomère oxo-6 moins polaire In) et 0,50 g (3,1 mmol) d'acide paratoluènesulfonique monohydrate sont mis dans 160 mL de toluène sont chauffés à reflux dans un appareil de Dean-Stark. Après 30 min de reflux, le milieu réactionnel est refroidi, dilué par 150 mL de chlorure de méthylène et agité en présence de 60 g de $Al_2O_3$ pendant 10 min. On filtre et le filtrat est concentré à 1/2 volume puis lavé à l'eau, séché et évaporé. Le résidu est repris par de l'acétone et le chlorhydrate est obtenu par addition qoutte à goutte d'éthanol HCl, jusqu'à pH6. Les cristaux sont filtrés, rincés à l'acétone et séchés sous vide. On obtient 4,37 g de chlorhydrate du produit désiré.

Fusion (chlorhydrate): 204°-205° C

Spectre RMN du produit base ($CDCl_3$, ppm) 7,5-6,9 (massif 4H), 6,8 (doublet 1H - J5,6: 6 cps), 5,2 (double doublet J56: 8 cps J 5,4 7 cps) 3,4 (doublet 1H J 4,3a 6 cps), 1,3-0,7 (massif 6H).

Spectre IR du produit de base (film, $cm^{-1}$) 2800, 1640, 1450, 1400, 1310, 1070, 910, 740.

Spectre UV du produit chlorhydrate (c = $3{,}32.10^{-5}$ mol/L MeOH, $\lambda$ max (log $\varepsilon$) 221 (4,40), 257(4,43), 301(3,86), 310(3,87) nm.

Spectre de masse M+: 266 pour $C_{18}H_{22}N_2$.

**Exemple 18**

Diéthyl-3,4-hexahydro-2,3,3a,4,5,6-hydroxy-6- 1H indolo (3,2,1-de) (1,5) naphtyridine-6-carboxylate de méthyle (Ir).

10 g. (0,03 mol) de diethyl-3,4- octahydro,-1,2,3,3a,4,5,6,7- oxo-7-oxyimino-6- azépino (1,2,3-lm)-β-carboline sont mis en suspension dans un mélange de 200 ml d'eau, 100 ml de MeOH et 1 00 ml de dioxane. On ajoute 9,4 g (0,09 mol) de $NaHSO_3$ et on chauffe à reflux. Après 6 h, 9,4 g (0,09 mol de $NaHSO_3$) sont encore ajoutés et le reflux est pour suivi pendant 16 h supplémentaires. Le milieu réactionnel est alors refroidis, dilué par 200 mL d'eau, basifié par une solution de $NH_4OH$ et extrait au $CH_2Cl_2$. La phase chlorure de méthylène est ensuite extraite par une solution diluée d'acide sulfurique à 2 %, et la phase aqueuse acide est finalement basifiée et extraite au $CH_2Cl_2$. Le chlorure de méthylène est lavé, séché et évaporé. On obtient 3,5 g de mélange de 2 isomères (Ir(a) et Ir(b), "petite vincamine" et "petite iso-vincamine") qui peuvent être utilisés pour l'exemple suivant.

Cependant lorsque le mélange de 2 isomères est dissous dans l'acétone et on ajoute du méthanol saturé en acide chlorhydrique, l'isomère le moins polaire Ir(a) cristallise et est isolé.

Fusion du produit chlorhydrate 213°C.

Spectre RMN du produit base ($CDCl_3$) 7,4-6,8 ppm (massif 4H), 4,4 ppm (massif 1H s'échange avec $D_2O$) 3,8 ppm (singulet 3h), 1,3-0,9 ppm (massif 6H)

Spectre IR du produit chlorhydrate: (KBr) $cm^{-1}$: 3300, 2960, 2550, 1740, 1460, 1230, 750.

Spectre UV du produit chlorhydrate (c = 2,64, $10^{-5}$ mol/L) max (log $\varepsilon$): 225(4,5), 272 (3,91), 275-279(3,90), 288-290 (3,75) nm.

Spectre de masse: M+ 342 pour $C_{20}H_{26}N_2O_3$.

**Exemple 19**

Diéthyl -3,4- tétrahydro- 2,3,3a,4 -1H indolo (3,2,1-de) (1,5) naphtyridine-6-carboxylate de méthyle (Is)

4,28 g (12,5 mmol) du mélange de 2 isomères de diéthyl-3,4- hexahydro -2,3,3a,4,5,6- hydroxy-6 1H indolo (3,2,1-de) (1,5) naphtyridine-6-carboxylate de méthyle sont dissous dans 100 mL de 1,2-dichloréthane. On ajoute 7,13 g (37,48 mmol) d'acide p-toluène sulfonique monohydrate et on distille l'azéotrope. Lorsque la température de distillation atteint 82°C on refroidit le milieu réactionnel. On ajoute 100 mL de chlorure de méthylène et on lave la solution par une solution aqueuse $NH_4OH$ puis à l'eau. La phase organique est alors agitée en présence de 25 g de $Al_2O_3$ et après filtration, le filtrat est lavé, séché et évaporé sous vide.

On obtient 3,7 g de produit désiré qui est dissous dans l'acétone et on ajoute du HCl gazeux jusqu'à pH acide. Les cristaux sont filtrés, rincés à l'acétone et séchés.

Fusion (chlorhydrate): 219 - 220°C.

Spectre RMN du produit base ($CDCl_3$): 7,5-6,9 (massif 4 H), 6,4 (doublet 1H), 3,9 (singulet 3H), 3,5 (doublet mal défini J : 5 cps) 1,1 (triplet: 3H) 0,75 (massif 3H).

Spectre IR du produit base (film, cm-1) 2960, 1725, 1640, 1610, 1450, 1280, 1260, 910, 740.

Spectre UV du produit chlorhydrate: (c = $2{,}76..10^{-5}$ mol/L MeOH) max (log $\varepsilon$) 228 (4,51), 274 (4,04), 314 (3,73) nm.

Spectre de masse: 324, 309, 295, 282, 269, 252, 238, 219, 198, 183, 169, 142.

**Exemple 20**

Diéthyl-3,4-octahydro-1,2,3,3a,4,5,6,7-oxo-7-oxyimino-6-azépino(1,2,3-lm)-β -carboline (III').

En suivant un mode opératoire identique à celui décrit dans l'exemple 14, à partir de 13,5 g de II dans lequel $R_1 = R_3 =$ éthyle et $R_2 = H$ (isomère le moins polaire en ccm) on obtient 12,3 g du dérivé oxyimino correspondant (III').

Fusion (chlorhydrate): 264,5° C

Spectre RMN du produit base ($CDCl_3$): 8,4 ppm (massif 1H), 7,4-7 ppm (massif 3H), 3,5 ppm (doublet 1H: $J_{3a-4}$:11 cps), 1,3-0,9 ppm (massif 6 H).

Spectre IR du produit base (film, $cm^{-1}$) 3400, 2960, 1680, 1610, 1450, 1380, 1330, 1020, 750, 730.

Spectre UV du produit chlorhydrate: (c; $5,33.10^{-5}$ m/l MeOH) max (log ε): 258 (4,16), 313 (3,71) nm.

Spectre de masse: 325, 308, 296, 282, 268, 251, 226, 199, 198, 197, 169 pour C19 H23 N3 O2.

**Exemple 21**

Diéthyl-3,4 -hexahydro-2,3,3a,4,5,6- oxo-6 1H indolo (3.2,1-de)(1,5) naphtyridine (In') (composé pas compris par les revendications pareque inactif).

En suivant un mode opératoire identique à celui de l'exemple 16 à partir de 9,56 g de (III') on obtient 4,17 g du dérivé In'.

Le dérivé (In') se révèle plus polaire en ccm que le dérivé diastéréoisomère In de l'exemple 15.

Fusion (chlorhydrate): 242°-243° C.

Spectre RMN du produit base ($CDCl_3$) 8,4 ppm (massif 1H), 7,4-7,1 ppm (massif 3H) 3,7 ppm (doublet 1H: $J_{3a-4}$: 10 cps), 1,3-0,8 ppm (massif 6 H).

Spectre IR du produit base (film) ($cm^{-1}$) 2970, 1700, 1630, 1450, 1380, 1330, 1140, 910, 740

Spectre UV du produit HCl: (c= $3,13.10^{-5}$ mol/L MeOH) λ max (log ε) 242(4,30), 266(4,00) 297-305 (3,59).

Spectre de masse: 282, 281, 267, 253, 239, 226, 199, 198, 197, 196, 168 pour C18 H22 N2 O.

**Exemple 22**

Diéthyl-3,4-hexahydro-2,3,3a,4,5,6-hydroxy-6-1H-indolo(3,2,1-de) (1,5)naphtyridine (Io')

En utilisant un mode opératoire analogue à celui de l'exemple 17 on obtient Io' avec un rendement de 85%.

Fusion 129-130° C

Spectre IR (base, KBr, $cm^{-1}$): 3350, 2920, 1420, 1250, 1170, 1025, 720

Spectre UV (base c = $3,15.10^{-5}$M/L, MeOH λ max log ε): 228 (4,52), 275-282(4,02), 290 (épaulement, 3,76).

Spectre de masse M + 264 pour $C_{18}H_{24}N_2O$.

Spectre RMN ($CDCl_3$ + $CD_3OD$): 7,4-6,9 (massif, 4H), 5,8 (massif,) 1H) 1,3-0,8 (massif, 6H).

**Exemple 23**

Diéthyl-3,4-tétrahydro-1,2,3,3a-4H-indolo(3,2,1-de)(1,5)naphtyridine (Ip')

En utilisant un mode opératoire analogue à celui de l'exemple 18 on obtient (Ip') avec un rendement de 69%.

Fusion (chlorhydrate) 199-201° C

Spectre IR (base, film, $cm^{-1}$) 2960, 1670, 1460, 1430, 910, 740.

Spectre UV (chlorhydrate, c = 3,31 $10^{-5}$ mol/L MeOH, λmax, log ε): 221 (4,40) 257 (4,45) 301 (3,89), 310 (3,89).

Spectre de masse: m/e 266 251 237 221 208, 194, 180, 167, 133 pour C18 H22 N2.

Spectre RMN (base, $CDCl_3$): 7,6-7 (massif 4H), 6,8 (1H double doublet, J5,6: 8cps, J5,4: 2cps), 3,8 (d, J4,3a: 12 cps), 1,2-0,6 (massif, 6H).

**Exemple 24**

Diéthyl-3,4-hexahydro -2,3,3a,4,5,6- hydroxy -6- 1H indolo (3,2,1-de)(1,5) naphtyridine-6-carboxylate de méthyle (Ir') (a et b).

11 g (33 8 mmol) de diéthyl-3,4-octahydro-1,2,3,3a,4,5,6,7-oxo-7-oxy-imino-6-azépino (1,2,3-lm)- β-carboline (III') (exemple 20) sont mis en suspension dans 200 mL d'eau, 100 mL de MeOH et 100 mL de dioxanne. On ajoute 17 g (0,14 mol) de $NaHSO_3$ et on chauffe à reflux. Après 5 h on ajoute à nouveau 17 g de $NaHSO_3$ et on chauffe à reflux pendant 16 h. Le milieu est ensuite refroidi, basifié et extrait au dichlorométriane. Le dichlorométhane est extrait par une solution d'acide sulfurique à 2% et la phase aqueuse décantée est basifiée

par une solution de $NH_4OH$ à 10 % et extraite au dichlorométhane. Le dichlorométhane est lavé, séché et évaporé sous pression réduite. On obtient ainsi 4 g de mélange de 2 isomères sous la forme d'un solide jaune qui peut être utilisé dans les étapes suivantes. On peut cependant séparer les deux isomères en position 6 ("petite vincamine" et "petite isovincamine") par des recristallisations successives du mélange dans l'acétone, l'acétate d'éthyle et l'éther. Le chlorhydrate de chacun des isomères peut être obtenu par dissolution du produit dans l'acétone et addition de méthanol saturé en HCl.

Caractéristiques physiques de l'isomère le moins polaire Ir'(a).

Fusion (base): 187° C.

Spectre RMN du produit base ($CDCl_3$ + $CD_3OH$): 7,4-6,8 ppm (massif 4H), 3,8 ppm (singulet 3 H), 1,3-0,9 ppm (massif 6H).

Spectre UV du produit base: (c = $2{,}97.10^{-5}$ mol/L MeOH). max (log ε) 226 (4,55), 276-280 (3,92), 288 épaulement nm.

Spectre IR du produit base (KBr, $cm^{-1}$) 2940, 1740, 1460, 1260, 1220, 1060, 750.

Spectre de masse: M+ 342 pour $C_{20}H_{26}N_2O_3$.

Caractéristiques physiques de l'isomère le plus polaire Ir'(b).

Fusion (chlorhydrate): 183° C.

Spectre RMN du produit base ($CDCl_3$) 7,4-6,9 ppm (massif 4H), 4,7. ppm (massif 1H s'échange avec $D_2O$), 3,6 ppm (singulet 3H), 1,2-0,8 ppm (massif 6H).

Spectre IR du produit base (KBr, $cm^{-1}$).. 3400, 2960, 1740, 1450, 1260, 1200, 1140, 1100, 750.

Spectre UV du produit chlorhydrate (c = $2{,}64.10^{-5}$ mol/l MeOH), max (log ε): 224(4,41), 273(3,79) 277-261(3,77), 288-290 (3,59) nm.

Spectre de masse M+ 342 pour $C_{20}H_{26}N_2O_3$.

**Exemple 25**

Diéthyl-3,4-tétrahydro-2,3,3a,4-1H-indolo (3,2,1-de)(1,5) naphtyridine -6-carboxylate de méthyle (I's)

12,62 g (37,4 mmol) du mélange des 2 isomères d'hexahydro-1,2,3,3a,4,5- diéthyl-3,4 -hydroxy-6-1H indolo (3,2,1-de) (1,5) naphtyridine-6-carboxylate de méthyle (Ir'(a) et Ir'(b) de l'exemple 24) sont dissous dans 30 mL de 1,2-dichloréthane. On ajoute 21,36 g (0,1 12 mol) d'acide p-toluène sulfonique monohydraté et on distille l'azéotrope.

Lorsque la température de distillation atteint 82° C on refroidit le milieu réactionnel.

On ajoute 300 ml de chlorure de méthyléne et on lave la solution par une solution diluée de $NH_4OH$ puis à l'eau.

La phase organique est alors agitée en présence de 64 g de $Al_2O_3$ et après filtration, le filtrat est lavé, séché et évaporé. Le résidu est dissous dans 100 mL d'acétone et on ajoute du HCl gazeux jusqu'à pH acide. Les cristaux sont filtrés, rincés à l'acétoné et séchés, on obtient 10,9 g de cristaux blancs.

Caractéristiques physiques:

Fusion du produit chlorhydrate: 199-200° C

Spectre RMN du produit base: ($CDCl_3$) 7,5-6,9 ppm (massif 4H), 6,2 ppm (doublet 1H J : 2 cps), 3,9 ppm (singulet 3H), 3,6 ppm (doublet J : 11 cps), 1,3-0,9 ppm (massif 6H).

Spectre IR du produit base (film, $cm^{-1}$): 2970, 1725, 1630, 1450, 1230, 910, 740.

Spectre UV du produit chlorhydrate (c : $2{,}77.10^{-5}$ mmol/l MeOH, max (log ε): 244(4,53), 269(4,09), 313(3,63) nm.

Spectre de masse (m/e): 324, 309, 307, 293, 238, 236, 180, 165, 127 pour C20 H24 N2 O2.

**Exemple 26**

(-)éthyle-4-hexahydro-1,2,3,3a,4,5-propyl-4-canthinone-6 (It).

a) 10 g (34 mmol) de déhydro-14,15 vincamone sont dissous dans 500 mL de méthanol et on y ajoute un excès d'iodure de méthyle soit 18,75 mL (42,8 g, 0,3 mol). Le mélange réactionnel est agité pendant 20 heures jusqu'à disparition complète en ccm de la déhydrovincamone. On évapore à sec et on cristallise dans le dichlorométhane. On isole ainsi 7,18 g d'ammonium quaternaire très polaire en ccm.

b) 7,18 g de l'ammonium quaternaire susmentionné sont mis en suspension dans le méthanol (115 mL) et on y ajoute 1,43 g de PtO2 80% après avoir saturé le réacteur avec de l'argon. L'appareillage est mis sous pression d'hydrogène après avoir chassé le gaz inerte et on laisse agiter sous pression ambiante pendant 20 h. La quantité théorique d'hydrogène étant absorbée, une analyse par ccm confirme la disparition du produit polaire. On filtre sur décalite et on évapore le solvant sous vide. Le résidu est repris avec du dichlorométhane et traité avec de l'eau ammoniacale. La phase organique est lavée encore deux fois à l'eau, séchée sur MgSO4 et évaporée à sec. On obtient ainsi 4,5 g de produit unitache que l'on peut recristalliser sous forme chrohydrate en présence de HCl-acétone-éther.

Spectre de résonance magnétique nucléaire (ppm CDCl3): 2,63 (s,3H), 3,5 (s,1H), 7,3 (m,3H), 6,3 (m,1H)

Spectre ultra-violet (méthanol, log ε): 242, 267, 294, 301 nm

Spectre infra-rouge ($CHCl3$, $cm^{-1}$): 3680, 3600, 2960, 2800, 1700, 1625, 1450, 1360, 1330
Pouvoir rotatoire $\alpha_D$ = -25,7° (c = 0,694, $CHCl3$)

Comme mentionné auparavant, l'invention comprend également les applications industrielles et notamment pharmaceutiques des produits décrits ci-dessus. Les composés de formule (I) ont été soumis à des essais pharmacologiques qui ont révélés des propriétés intéressantes notamment des propriétés antianoxiques, psychotropes et oxygénatrices cérébrales.

**Toxicité aiguë:**

Les composés de l'invention ont été administrés sous forme de chlorhydrate par voie intraveineuse à des souris de souche Charles River. Les doses létales 50 % ($DL_{50}$) ont été déterminées g raphiquement sel on la méthode de Lichtfield et Wilcoxon (J. Pharmacol. Exp. Thérap. 1946, 96, 99).

**Epreuve d'anoxie hypobare chez la souris:**

Des souris de même sexe, de souche Charles River, pesant 20 +/- 2g sont réparties en trois lots de 10 animaux. Les lots 1 et 2 comportent les animaux traités, c'est-à-dire ayant reçu la substance à tester de l'invention sous forme chlorhydrate. Un troisième lot comporte des animaux témoins.

Les composés étudiés sont administrés p.o 30 minutes avant l'essai. Les animaux sont placés dans une atmosphère appauvrie en oxygène par réalisation d'un vide partiel (190 mm Hg, correspondant à 5,25 % d'oxygène), ces conditions étant obtenues en 30 secondes. On mesure le temps de survie des souris au moyen d'un chronomètre. Les agents capables de favoriser l'oxygénation tissulaire et en particulier cérébrale, entraînent un accroissement du temps de survie et l'on peut exprimer par la dose efficace 50 % ($DE_{50}$), la dose qui augmente de 50 % le temps de survie moyen des animaux placés dans les conditions décrites ci-dessus.

Les résultats obtenus avec quelques dérivés de l'invention sont rassemblés dans le tableau suivant:

| Dérivé | Exemple | Toxicité aigue $DL_{50}$ | | Hypoxie hypobare | |
|---|---|---|---|---|---|
| | n° | i.g. | i.v. | $DE_{50}$ | Coeff |
| Ig | 8 | 2000 | 82 | 117 | 17,9 |
| In' | 21 | 550 | 11 | inactif | |
| In | 15 | 1500 | 65 | 45 | 33 |
| Ik | 12 | 1425 | 70 | 40 | 35,6 |
| Ie | 6 | 545 | 32 | | |
| vincamine | | 1150 | 46 | 90 | 12,7 |
| vincamone | | 796 | 30 | 25 | 31,8 |
| Is | 19 | 2000 | | 92 | 22 |
| Ip | 17 | 860 | | 70 | 12,3 |
| Is' | 25 | 1585 | | 95 | 16,6 |
| Ip' | 23 | 220 | | 40 | 5,5 |

Comparés à la vincamine, à fraction de $DL_{50}$ identique, la plupart des dérivés de la présente invention sont donc plus actifs dans le test d'hypoxie hypobare. On relèvera en particulier l'activité exceptionnelle du composé In, alors que l'isomère In' n'est pas actif.

Les composés correspondants aux structures In, Is et Ip sans groupe éthyle en position 4 de la formule 1 ont des coefficient LD50/DE50 hypoxie hypobare respectivement de 10,3, 5,5, et 12,3. Les composés de l'invention se comparent donc avantageusement à ceux déjà décrits dans l'état de la technique.

La (+/-)diéthyl-3,4-hexahydrocanthinone-6 In et le dérivé carbométhoxy-6 Is'présentent également des propriétés hémodynamiques et oxymétriques exceptionnelles, comme l'indique entre autres l'augmentation de la consommation d'oxygène mesurée par la $CMRO_2$ (cerebral metabolic rate of $O_2$).

D'autres tests pharmacologiques démontrent la supériorité des composé de l'invention comparés à ceux possédant une structure similaire connue, en particulier les dérivés correspondants ne possédant pas des groupe alkyle en position 4 de la formule (I).

On a aussi démontré que la cardiotoxicité des dérivés In et Ig comparée à celle de la vincamine, de la vincamone et de la vinpocétine est bien moins importante. Ainsi 20 mg i.v. de In chez le lapin est moins cardiotoxique que 1 mg de vincamine.

D'autre part le test de l'action contre l'oedème cérébral induite par le triéthyl-étain (J. Path. Bact. 73, 107-123, 1957) s'est révélé particulièrement positif par exemple pour les composés (Ig), (In), (Is) et (Ip) qui sont tous plus actifs que la vincamine. Is à peu près quatre fois supérieure à la vincamine.

Les composés de l'invention, possédant à la fois une activité antianoxique et une activité psychotrope, peuvent être utilisés en thérapeutique pour le traitement des troubles de la vigilance, en particulier pour lutter contre les troubles du comportement imputables à des dommages vasculaires cérébraux et à la sclérose cérébrale en gériatrie, comme sédatif, ainsi que pour le traitement des absences dues à des traumatismes crâniens, et le traitement des états dépressifs.

On peut également les utiliser de manière générale pour le traitement des affections cérébro-vasculaires ou cardiocirculatoires.

Pour leur application en thérapeutique, les composés de l'invention peuvent être administrés soit par voie digestive sous forme de capsules, gélules, comprimés, dragées, cachets, solution ou suspension, soit par voie parentérale sous forme de soluté stérile tamponné préparé à l'avance ou extemporanément, dans lesquels la substance active, à l'état de base ou salifiée, se trouve présente à raison de 0,5 mg à 700 mg de substance active par dose unitaire.

L'invention se réfère éqalement spécifiguement aux médicament sous la forme de comprimés, de gélules on de solutés injectables comportant la (+/-) diéthyle-3.4-hexahydro-2,3,3a,4,5,6-oxo-6-1H-indolo (3.2.1-de) (1.5)-naphtyridine ou son sel d'addition avec l'acide chlorhydrique, dosés unitairement de 1 à 500 mg de produit actif.

La posologie quotidienne peut varier entre 1 mg et 700 mg de substance active selon l'affection.

En vue de leur application thérapeutique, les composés de l'invention sont présentés sous forme de compositions pharmaceutiques contenant comme substance active au moins un composé selon l'invention, éventuellement en mélange avec d'autres substances actives et les adjuvants, diluants, véhicules ou excipients habituels en pharmacie ainsi que colorants, édulcorants, agents de conservation anti-oxydants, etc...

La préparation pharmacologique ou galénique s'effectue par les méthodes classiques, essentiellement par incorporation ou mélange des substances actives dans les adjuvants utilisés.

Dans le cas où les composés sont administrés sous forme de leurs sels d'addition, il convient d'utiliser des acides pharmaceutiquement admissibles qui sont bien connus en pharmacie, en particulier l'acide chlorhydrique, l'acide sulfurique et l'acide méthanesulfonique.

**Revendications**

1. A titre de composés nouveaux les dérivés d'hexahydro-2,3,3a,4,5,6-1H-indolo(3,2,1-de)(1,5)naphtyridine de formule (I)

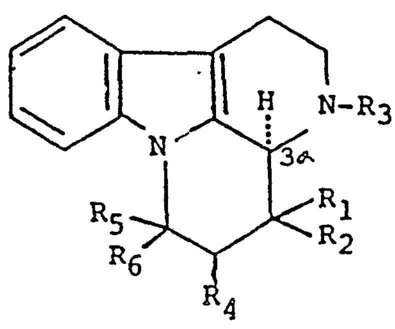

sous forme de base ou de sel d'addition d'acide, de préférence d'acide pharmaceutiquement acceptable et éventuellement sous la forme d'isomères optiques, dans laquelle

l'un des groupes $R_1$ et $R_2$ représente un groupe alkyle inférieur comportant 1 à 3 atomes de C et l'autre représente un atome d'hydrogène en position "cis" par rapport à l'hydrogène 3a ou, $R_1$ et $R_2$ représentent indépendamment un groupe alkyle comportant 1 à 3 atomes de C ou ensemble un groupe alkanediyl comportant de 4 à 6 atomes de carbone,

$R_3$ représente un groupe alkyle comportant 1 à 3 atomes de C, un atome d'hydrogène ou un groupe benzyle et soit,

$R_5$ représente un groupe carboalkoxy comportant 2 à 4 atomes de C ou un atome d'hydrogène et $R_6$ représente avec $R_4$ une liaison carbone-carbone supplémentaire soit

· $R_4$ représente un atome d'hydrogéne et $R_6$ et R5 représentent ensemble un atome d'oxygène ou respectivement un atome d'hydrogène et un groupe hydroxy.

2. Composés selon la revendications 1 caractérisé en ce que dans la formule I $R_1$ et/ou $R_2$ représentent des groupes éthyle.

3. Composés selon la revendication 1 caractérisé en ce que $R_1=R_3=$éthyle, $R_2=R_4=H$ et $R_5$ et $R_6$ représentent ensemble un atome d'oxygène.

4. Composé selon la revendication 3 caractérisé en ce qu'il s'agit du diastéréoisomère le moins polaire en chromatographie sur couche mince de silica gel, l'éluant étant un mélange dichlorométhane-méthanol, (98:2, v:v).

5. Composés selon les revendications 1 ou 2 caractérisés en ce que $R_1$ = H et $R_2$ = bêta-éthyle.

6. La (+/-) diéthyl- 3,4- hexahydro- 2,3.3a,4,5.6- oxo- 6 -1H- indolo (3,2,1-de) (1,5) - naphtyridine et son sel d'addition avec l'acide chlorhydrique.

7. Composé selon la revendication 1 caractérisé en ce qu'it s'agit d'un des composés suivants:

- (+/-) diéthyl -3,4- hexahydro -2,3,3a,4,5,6- hydroxy-6-1H-indolo (3,2,1-de) (1,5) naphtyridine
- (+/-) triéthyl-3,4,4 -hexahydro-2,3,3a,4,5,6 -oxo-6-1H-indolo (3,2,1-de) (1,5) naphtyridine
- (+/-) diéthyl-3,4 -tétrahydro-2,3,3a,4- 1H -indolo (3,2,1-de) (1,5) naphtyridine-6-carboxylate de méthyle
- (+/-) diéthyl -3,4- tétrahydro-2,3,3a,4 -1H-indolo (3,2,1-de)(1;5) naphtyridine.
- (+/-) ethyl -3- spirocyclohexyl -4- tétrahydro-2,3,3a,4 -1H-indolo (3,2,1-de) (1,5) naphtyridine-6-carboxylate de méthyle
- (+/-) ethyl -3- spirocyclohexyl -4- hexahydro -2,3,3a,4,5,6-oxo-6-1 H- indolo (3,2,1-de) (1,5)naphtyridine

et leurs sels d'addition avec des acides pharmaceutiquement acceptables.

8. Composés selon la revendication 2 caractérisés en ce que R1 représente un groupe éthyle et R2 représente un atome d'hydrogène.

9. Composés selon la revendication 6 caracterisés en ce que les hydrogènes 3a et 4 sont en position relative de configuration cis.

10. Médicaments pour le traitement des affections résultant d'une insuffisance d'oxygénation cérébrale, caractérisés en ce qu'ils comprennent un des composés définis dans les revendications 1 à 5 soit sous forme de base, soit sous la forme de sel d'addition d'acide mingraux ou organiques pharmaceutiquement acceptables.

11. Médicaments sous la forme de comprimés, de gélules ou de solutés injectables, comportant, à titre de produit actif, le composé de la revendication 6, dosés unitairement de 1 à 500 mg de produit actif.

## Patentansprüche

1. Als neue Verbindungen, Derivate des Hexahydro-2 3 3a 4 5 6, -1H- indolo (3,2,1-de)(1,5) naphtyridins der Formel

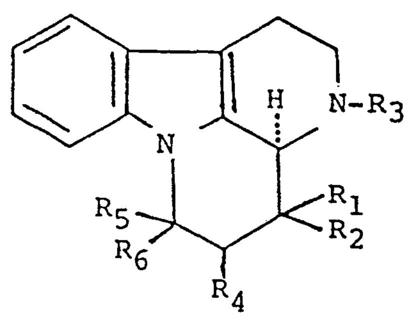

(I)

in der Form der Base oder des Additionssalzes, vorzugsweise mit einer pharmazeutisch annehmbaren Säure, und gegebenenfalls in der Form von optischen Isomeren, wobei eine der Gruppen $R_1$ und $R_2$ eine niedrige Alkylgruppe mit 1-3 C-Atomen bedeutet und die andere ein Wasserstoffatom in der "cis"-Stellung im Verhältnis zum 3a-Wasserstoff-oder $R_1$ und $R_2$ unabhängig eine Alkylgruppe mit 1-3 C-Atomen oder zusammen eine Alkandiylgruppe mit 4 bis 6 C-Atomen darstellen,

$R_3$ eine Alkylgruppe mit 1-3 C-Atomen, ein Wasserstoffatom oder eine Benzylgruppe darstellt, und entweder R5 eine Carboalkoxygruppe mit 2-4 C-Atomen oder ein Wasserstoffatom bedeutet und $R_6$ mit $R_4$ eine zusätzliche Kohlenstoff-Kohlenstoff-Bindung darstellt, oder

$R_4$ ein Wasserstoffatom und $R_6$ und $R_5$ zusammen ein Sauerstoffatom bedeuten oder jeweils ein Wasserstoffatom und eine Hydroxygruppe darstellen.

2. Verbindungen nach Anspruch 1 dadurch gekennzeichnet, daß in der Formel I, $R_1$ und/oder $R_2$ Athylgruppen darstellen.

3. Verbindungen nach Anspruch 1 dadurch gekennzeichnet, daß $R_1$ = $R_3$ = Äthyl, $R_2$ = $R_4$ = H und $R_5$ und $R_6$ zusammen ein Sauerstoffatom darstellen.

4. Verbindung nach Anspruch 3 dadurch gekennzeichnet daß es sich um das am wenigsten polarisierten Diastereoisomer in Dünnschichtchromatographie auf Silica Gel handelt, wobei das Eluiermittel aus einer Mischung Dichloromethan-Methanol (95:2, V:V) besteht.

5. Verbindungen nach den Ansprüchen 1 oder 2 dadurch gekennzeichnet, daß $R_1 = H$ und $R_2$=beta-Äthyl.

6. (+/-) Diäthyl-3,4- hexahydro-2,3,3a,4,5,6-oxo-6 -1H- indolo (3,2,1-de) (1,5) -naphtyridin und sein Additionssalz mit der Salzsäure.

7. Verbindung nach Anspruch 1 dadurch gekennzeichnet, daß es sich um eine der folgenden Verbindungen handelt:

- (+/-) Diäthyl-3,4- hexahydro-2,3,3a,4,5,6- hydroxy-6-1H-indolo (3,2,1-de)(1,5) naphtyridin
- (+/-) Triäthyl-3,4,4 -hexahydro-2,3,3a,4,5,6-oxo-6-1H-indolo (3,2,1-de)(1,5) naphtyridin
- Methyl (+/-) Diäthyl-3,4 -tetrahydro-2,3,3a,4-1H -indolo (3,2,1-de)(1,5) naphtyridin-6-carboxylat
- (+/-) Diäthyl-3,4 -tetrahydro-2,3,3a,4- 1H -indolo (3,2,1-de) (1,5) naphtyridin
- Methyl (+/-) Äthyl -3- spirocyclohexyl -4- tetrahy-dro-2,3,3a,a -1H-indolo (3,2,1-de)(1,5) naphtyridin-6-carboxylat
- (+/-) Äthyl -3- spirocyclohexyl -4- hexahydro -2,3,3a,4,5,6- oxo-6-1H- indolo (3,2,1-de)(1,5) naphtyridin

und deren Additionssalze mit pharmazeutisch annehmbaren Säuren.

8. Verbindungen nach Anspruch 2 dadurch gekennzeichnet, daß $R_1$ eine Äthylgruppe und $R_2$ ein Wasserstoffatom darstellt.

9. Verbindungen nach Anspruch 6 dadurch gekennzeichnet, daß die Wasserstoffatome in 3a- und 4-Stellung relativ zueinander in der "cis"-Konfiguration stehen.

10. Arzneimittel zur Behandlung von einer aus einer ungenügenden zerebralen Sauerstoffzuführung rührenden Erkrankung, dadurch gekennzeichnet, daß sie eine der Verbindungen der Ansprüche 1 bis 5 enthalten, entweder in der Form der Base oder in der Form des Additionssalzes mit einer anorganischen oder organischen pharmazeutisch annehmbaren Säure.

11. Arzneimittel in Form von Tabletten, Gelkapseln oder injizierbaren Lösungen, die als Wirkstoff die Verbindung aus Anspruch 6 enthalten, und einheitlich mit 1 bis 500 mg Wirkstoff dosiert sind.

## Claims

1. As new compounds, the 2,3,3a,4,5,6-hexahydro 1H indolo (3,2,1-de)(1,5) naphtyridine derivatives of formula (I)

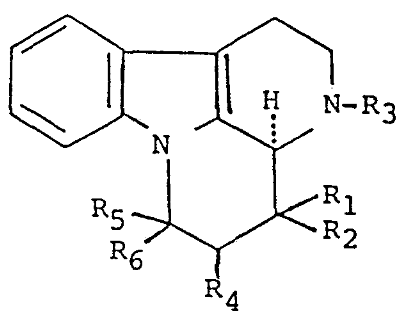

(I)

in the form of base or acid addition salt, preferably of pharmaceutically acceptable acid, and possibly in the form of optical isomers, wherein one of the groups Rl and R2 represents a lower alkyl group with 1 to 3 C-atoms and the other represents a hydrogen atom in "cis" position with reference to the 3a hydrogen or R1 and R2 represent each independently an alkyl group with 1 to 3 C-atoms or, together, an alkanediyl group having from 4 to 6 carbon atoms,

R3 represents an alkyl group with 1 to 3 C-atoms, a hydrogen atom or a benzyl group and either

R5 represents a carboalkoxy group with 2 to 4 C-atoms or a hydrogen atom and R6 represents with R4 an additional carbon-carbon bond

or

R4 represents a hydrogen atom and R6 and R5 represent together an oxygen atom or respectively a hydrogen atom and a hydroxy group.

2. Compounds according to claim 1 characterized in that in formula I Rl and/or R2 represent ethyl groups.

3. Compounds according to claim 1 characterized in that R1 = R3 = ethyl, R2 = R4 = H and R5 and R6 represent together an oxygen atom.

4. Compounds according to claim 3 characterized in that it is the less polar isomer as assessed by thin layer chromatography on silica gel, the eluent being a mixture dichloromethane-methanol (95:2, v:v).

5. Compounds according to claim 1 or 2 characterized in that R1 = H and R2 = beta-ethyl.

6. (+/-) 3,4-diethyl -2,3,3a,4,5,6-hexahydro -6-oxo -1H- indolo (3,2,1-de) (1,5) naphtyridine and its addition salt

with hydrochloric acid.

7. Compound according to claim 1 characterized in that it is one of the following compounds:
- (+/-) 3,4-diethyl -2,3,3a,4,5,6-hexahydro -6-hydroxy -1H-indolo (3,2,1)(1,5) naphtyridine
- (+/-) 3,4,4-triethyl -2,3,3a,4,5,6-hexahydro 6-oxo -1H-indolo (3,2,1-de)(1,5) naphtyridine
- (+/-) methyl 3,4-diethyl -2,3,3a,4-tetrahydro -1H-in-dolo (3,2,1-de)(1,5) naphtyridine-6-carboxylate
- (+/-) 3,4-diethyl -2,3,3a,4,-tetrahydro -1H-indolo (3,2,1-de)(1,5) naphtyridine
- (+/-) methyl 3-ethyl- 4 -spirocyclohexyl - 2,3,3a,4-tetrahydro IH indolo (3,2,1-de)(1,5) naphtyridine-6-carboxylate
- (+/-) 3-ethyl -4-spirocyclohexyl -2,3,3a,4,5,6-hexahy -dro -6-oxo -1H- indolo (3,2,1-de)(1,5) naphtyridine

and their addition salts with pharmaceutically acceptable acids.

8. Compounds according to claim 2 caracterized in that R1 represents an ethyl group and R2 is a hydrogen atom.

9. Compounds according to claim 6 characterized in that the hydrogens 3a and 4 are in a relative position having the cis configuration.

10. Drugs for the treatment of disorders resulting from insufficient cerebral oxygenation, characterized in that they comprise at least one of the compounds as defined in claims 1 to 5, in the form of a base or a pharmaceutically acceptable organic or inorganic acid addition salt thereof.

11. Drugs in the form of tablets, capsules or injectable solutes comprising as active compound the compound of claim 6 in unitary doses of from 1 to 500 mg of the active compound.